# EUROPÄISCHE PATENTSCHRIFT

(11) **EP 0 769 016 B1**
(45) Veröffentlichungstag und Bekanntmachung des Hinweises auf die Patenterteilung: **30.09.1998**
(21) Anmeldenummer: 95924330.4
(22) Anmeldetag: 28.06.1995
(51) Int. Cl.: C07H 15/04, C07C 303/24, C07C 303/44, B01F 17/08, B01F 17/56

(54) **VERFAHREN ZUR HERSTELLUNG HELLFARBIGER TENSIDE**
PROCESS FOR PRODUCING LIGHT-COLOURED SURFACTANTS
PROCEDE DE PRODUCTION D'AGENTS TENSIOACTIFS DE COULEUR CLAIRE

(30) Priorität: 06.07.1994 DE 4423641
(43) Veröffentlichungstag der Anmeldung: 23.04.1997
(73) Patentinhaber: Henkel Kommanditgesellschaft auf Aktien, 40191 Düsseldorf (DE)
(72) Erfinder: WEUTHEN, Manfred, D-42697 Solingen (DE)
(86) Internationale Anmeldenummer: EP9502514
(87) Internationale Veröffentlichungsnummer: WO9601269

(56) Entgegenhaltungen:
- WO-A-93/13112
- WO-A-93/21196
- WO-A-94/02494
- WO-A-94/03423
- DE-A- 2 524 785
- GB-A- 2 274 105
- US-A- 4 871 423

## Beschreibung

### Gebiet der Erfindung

Die Erfindung betrifft ein Verfahren zur Herstellung hellfarbiger Tenside, bei dem man die Tenside vor der Bleiche durch Zugabe von Antioxidantien stabilisiert.

### Stand der Technik

Die meisten oberflächenaktiven Verbindungen vom Aniontensid-Typ, aber auch bestimmte nichtionische Tenside, wie beispielsweise Alkyloligoglykoside, sind herstellungsbedingt als Folge z. B. von Oxidations- und Kondensationsprozessen dunkel gefärbt. Obschon diese Verfärbungen die anwendungstechnischen Eigenschaften der Stoffe kaum oder nur in sehr geringem Maße nachteilig beeinflußen, kommen aus ästhetischen Gründen doch nur solche Stoffe für eine Weiterverarbeitung in Betracht, deren Farbe durch eine nachträgliche Bleichoperation deutlich aufgehellt worden ist. Üblicherweise werden hierfür Persauerstoffverbindungen, wie beispielsweise Wasserstoffperoxid, Perborate oder Percarbonate eingesetzt, die die früher häufig verwendeten Hypochlorite beinahe vollständig verdrängt haben.

In der Vergangenheit hat es nicht an Versuchen gemangelt, die trotz Bleiche vielfach nicht zufriedenstellende Farbqualität von Tensiden weiter zu verbessern. So wird beispielsweise in der **WO 93/13 113** (Henkel) vorgeschlagen, die Wirkung des Wasserstoffperoxids durch den Einsatz von verschiedenen Bleichboostern wie beispielsweise Magnesiumionen und dergleichen zu verstärken. In der Praxis hat sich zwar gezeigt, daß Produkte erhalten werden, die über eine deutlich verbesserte Farbe verfügen, hinsichtlich ihrer Lagerstabilität müssen jedoch auch weiterhin deutliche Abstriche hingenommen werden.

Die Aufgabe der Erfindung hat somit darin bestanden, diesem Mangel abzuhelfen und ein Verfahren zur Verfügung zu stellen, mit dessen Hilfe Produkte erhalten werden, die unmittelbar nach der Bleiche hell farbig sind und sich auch nach längerer Lagerung bei gegebenenfalls höheren Temperaturen als stabil erweisen.

### Beschreibung der Erfindung

Gegenstand der Erfindung ist ein Verfahren zur Herstellung hellfarbiger Tenside, das sich dadurch auszeichnet, daß man die Tenside vor der Bleiche durch Zugabe von Antioxidantien stabilisiert.

In einer bevorzugten Ausführungsform betrifft die Erfindung ein Verfahren zur Herstellung von hellfarbigen Alkyl- und/ oder Alkenyloligoglykosiden durch sauer katalysierte Acetalisierung von Zuckern mit Fettalkoholen, Neutralisation, destillatives Abtrennen überschüssigen Fettalkohols, Anpasten mit Wasser und alkalische Bleiche, das sich dadurch auszeichnet, daß man man die Glycoside vor der Bleiche durch Zugabe von Antioxidantien stabilisiert.

In einer weiteren bevorzugten Ausführungsform betrifft die Erfindung ein Verfahren zur Herstellung von hellfarbigen Alkyl- und/oder Alkenylsulfaten durch Sulfatierung von Fettalkoholen, Neutralisation mit wäßrigen Basen und Bleiche, das sich dadurch auszeichnet, daß man die Sulfate vor der Bleiche durch Zugabe von Antioxidantien stabilisiert.

Überraschenderweise wurde gefunden, daß der Zusatz bekannter Antioxidantien zu wäßrigen Tensidpasten diese während der Bleiche in einem solchen Maße stabilisieren, daß einerseits besonders farbhelle Produkte erhalten werden, die andererseits auch nach mehrwöchiger Lagerung bei erhöhter Temperatur praktisch nicht nachdunkeln. Die Erfindung schließt die Erkenntnis ein, daß der Zeitpunkt, an dem die Stabilisatoren den Tensiden zugegeben werden kritisch ist. Insbesondere eine nachträgliche Zugabe von Antioxidantien nach der Bleiche (also zum Verkaufsprodukt) erweist sich weder im Hinblick auf die Farbqualität noch auf die Lagerstabilität als vergleichbar. Die Tenside liegen vorzugsweise als wäßrige Lösungen oder Pasten mit Feststoffgehalten im Bereich von 5 bis 50 und vorzugsweise 15 bis 40 Gew.-% vor. Das erfindungsgemäße Verfahren kann jedoch grundsätzlich auch auf wasserfreie Systeme angewendet werden.

### Tenside

Das erfindungsgemäße Verfahren kann zur Farbverbesserung von anionischen, nichtionischen, kationischen und/oder ampholytischen bzw. zwitterionischen Tensiden eingesetzt werden.

Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Alkyloligoglucosidsulfate und Alkyl(ether)phosphate. Sofern die anionischen Tenside Polyglycoletherketten enthalten, können sie eine konventionelle, vorzugsweise jedoch eine eingeengte Homologenverteilung aufweisen.

Unter den anionischen Tensiden, auf die sich das erfindungsgemäße Verfahren bezieht, sind solche mit Sulfat- oder Sulfonatstruktur sowie Eiweißfettsäurekondensate bevorzugt.

Typische Beispiele für **nichtionische Tenside** sind Fettalkoholpolyglycolether, Alkylphenolpolyglycolether, Fettsäurepolyglycolester, Fettsäureamidpolygylcolether, Fettaminpolyglycolether, alkoxylierte Triglyceride, Alk(en)yloligoglykoside, Fettsäure-N-alkylglucamide, Polyolfettsäureester, Zukkerester, Sorbitanester und Polysorbate. Sofern die nichtionischen Tenside Polyglycoletherketten enthalten, können sie eine konventionelle, vorzugsweise jedoch eine eingeengte Homologenverteilung aufweisen.

Unter den nichtionischen Tensiden, auf die sich das erfindungsgemäße Verfahren bezieht, sind Alkylpolyglucoside, Methylglucosidester, Alkylglucosidester, Saccharoseester und Oligoglycerinester bevorzugt.

Typische Beispiele für **kationische Tenside** sind quartäre Ammoniumverbindungen und Esterquats, insbesondere quaternierte Fettsäuretrialkanolaminester-Salze.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine.

Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S.54-124 oder J.Falbe (ed.), "Katalysatoren, Tenside und Mineralöladditive", Thieme Verlag, Stuttgart, 1978, S.123-217 verwiesen.**

### Alkyl- und/oder Alkenyloligoglykoside

Alkyl- und Alkenyloligoglykoside stellen bekannte Stoffe dar, die der Formel **(I)** folgen,

**R**^{**1**}**O-[G]**_{**p**} **(I)**

in der R¹ für einen Alkyl- und/oder Alkenylrest mit 4 bis 22 Kohlenstoffatomen, G für einen Zuckerrest mit 5 oder 6 Kohlenstoffatomen und p für eine Zahl im Bereich von 1 bis 10 steht. Die Glykoside können nach den einschlägigen Verfahren der präparativen organischen Chemie erhalten werden. Stellvertretend für das umfangreiche Schrifttum sei hier auf die Schriften **EP-A1 0301298** und **WO 90/03977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligogl**ucoside.**

Die Indexzahl p in der allgemeinen Formel **(I)** gibt den Oligomerisierungsgrad (DP-Grad), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3).

Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalko- hol, Erucylalkohol, sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Alkyl- und/oder Alkenylsulfate

Unter Alkyl- und/oder Alkenylsulfaten sind die Sulfatierungsprodukte primärer Alkohole zu verstehen, die der Formel (II) folgen,

**R**^{**2**}**O-SO**_{**3**}**X (II)**

in der R² für einen linearen oder verzweigten, aliphatischen Alkyl- und/oder Alkenylrest mit 6 bis 22, vorzugsweise 12 bis 18 Kohlenstoffatomen und X für ein Alkyli- und/oder Erdalkalimetall, Ammonium, Alkylammonium, Alkanolammonium oder Glucammonium steht.

Typische Beispiele für Alkylsulfate, die im Sinne der Erfindung Anwendung finden können, sind die Sulfatierungsprodukte von Capronalkohol, Caprylalkohol, Caprinalkohol, 2-Ethylhexylalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technischen Gemischen, die durch Hochdruckhydrierung technischer Methylesterfraktionen oder Aldehyden aus der Roelen' schen Oxosynthese erhalten werden. Die Sulfatierungsprodukte können vorzugsweise in Form ihrer Alkalisalze, und insbesondere ihrer Natriumsalze eingesetzt werden. Besonders bevorzugt sind Alkylsulfate auf Basis von C_{16/18}-Talgfettalkoholen bzw. pflanzlicher Fettalkohole vergleichbarer C-Kettenverteilung in Form ihrer Natriumsalze.

### Antioxidantien

Unter Antioxidantien oder (Aut-)oxidationsinhibitoren werden in der Regel Stoffe verstanden, die unerwünschte, durch Sauerstoffeinwirkung und andere oxidative Prozesse bedingte Veränderungen in den zu stabilisierenden Stoffen verhindern oder zumindest hemmen. Die Wirkung von Antioxidantien besteht meist darin, daß sie als Radikalfänger für die bei der Autoxidation auftretenden freien Radikale wirken [vgl. **INFORM, 1,**, **1002 (1990)**]. Typische Beispiele für Antioxidantien sind:
*** Phenole und Phenolderivate wie beispielsweise tert. Butylhydroxytoluol (BHT), Di-tert.Butylhydroxytoluol (DBHT), Di-tert.Butylhydroxyanisol (BHA), Hydrochinon, Ethoxyquin, tert.Butylhydroxychinon, Di-tert.Butylhydroxychinon, Anoxomer, Gallussäure, Gallussäurester, Rosmarindiphenol, Boldine sowie α-, β- und γ-Tocopherol bzw. deren Acetate [vgl. **Fat Sci. Technol. 92, 201 (1990)**].
*** Hydroxycarbonsäuren, deren Salze und Alkylester wie beispielsweise Milchsäure, Äpfelsäure, Citronensäure, Weinsäure, Ascorbinsäure und Ancorbinpalmitat;
*** Flavenoide wie beispielsweise Catechin, Hespiridin, Morin, Naringin, Quercitin und Rutin [vgl. **J.Am.Oil.Chem. Soc. 70, 773 (1993)**];
*** Fettsäureamide, Pyridinverbindungen und Alkalinitrite.

Vorzugsweise werden Antioxidantien eingesetzt, die ausgewählt sind aus der Gruppe, die von Tocopherol, BHT, BHA, Ascorbinsäure, Ascorbinpalmitat, Citronensäure und/oder Alkalinitriten gebildet wird. Hierunter besonders bevorzugt ist der Einsatz von Tocopherolen. Üblicherweise können die Antioxidantien in Konzentrationen von 5 bis 2000, vorzugsweise 25 bis 1000 und insbesondere 50 bis 200 ppm - bezogen auf die Summe der Einsatzstoffe - eingesetzt werden. In einer bevorzugten Ausführungsform der Erfindung können die Antioxidantien mit bekannten Bleichboostern wie beispielsweise Magnesiumsalzen, Zeolithen, Hydrotalcit, Alkalalisilicaten und dergleichen kombiniert werden.

Werden Alkylglucosid-Pasten stabilisiert, können die Stabilisatoren den Pasten vor oder nach der Destillation des überschüssigen Fettalkohols zugesetzt werden. Im Falle von anionischen Tensiden mit Sulfat- und/oder Sulfonatstruktur kann der Zusatz vor oder nach der Neutralisation erfolgen. In beiden Fällen muß die Zugabe jedoch vor der Bleiche stattfinden.

### Gewerbliche Anwendbarkeit

Die nach dem erfindungsgemäßen Verfahren erhältlichen Tenside zeichnen sich durch eine besonders vorteilhafte Farbqualität aus und erweisen sich auch nach mehrwöchiger Lagerung bei erhöhter Temperatur stabil. Sie eignen sich zur Herstellung oberflächenaktiver Mittel wie beispielsweise Wasch-, Spül- und Reinigungsmittel, in denen sie in Mengen von 1 bis 50, vorzugsweise 5 bis 25 Gew.-% - bezogen auf die Mittel - enthalten sein können.

### Beispiele

### I. Herstellung einer Alkylpolyglucosid-Paste

In einer 5-l-Destillationsapparatur wurden 900 g (5 mol) wasserfreie Glucose vorgelegt und mit 1820 g (12,5 mol) einer technischen 1:1-Mischung von Octanol und Decanol (LOROL^{(R)} 810, Henkel KGaA, Düsseldorf/FRG) vermischt. Bei einer Temperatur von 100°C und einem verminderten Druck von 45 mbar wurden 10,4 g (0,004 mol) p-Toluolsulfonsäure in Form einer 65 Gew.-%igen wäßrigen Lösung zugetropft und das bei der Acetalisierung freiwerdende Wasser kontinuierlich aus dem Reaktionsgemiisch entfernt. Nach 8 h wurde der Ansatz auf 80°C abgekühlt und mit Stickstoff belüftet. Anschließend erfolgte die Neutralisation mit 1,5 ml (0,03 mol) 50 Gew.- %iger Natriumhydroxidlösung und 2 g (0,05 mol) Magnesiumoxid.

Nach 120 min wurde die Mischung mit dem Antioxidants versetzt (Variante 1) und der überschüssige Fettalkohol bei einer Temperatur von 160°C und einem verminderten Druck von 0,1 mbar abdestilliert.

Anschließend wurden 140 g des festen Reaktionsproduktes in 75 g Wasser bei 80°C gelöst. Die dunkelgefärbte Paste wurde mit dem Antioxidants (Variante 2) und 4 ml 50 Gew.-%iger Natriumhydroxidlösung versetzt. Bei einer Temperatur von 90°C wurden schließlich 5,5 ml Wasserstoffperoxid in Form einer 35 Gew.-%igen Lösung zugesetzt und über 4 h gerührt. Der pH-Wert wurde durch Zugabe von Natriumhydroxidlösung auf 11,5 eingestellt.

In Tabelle 1 sind die Farbzahlen nach Lagerung über 24 h sowie 2 bzw. 4 Wochen bei 70°C in Abhängigkeit von den eingesetzten Antioxidantien wiedergegeben. Für Vergleichsbeispiel V2 wurde Beispiel 1 wiederholt, Tocopherol jedoch erst nach der alkalischen Bleiche dem Verkaufsprodukt zugesetzt.

**Tabelle 1**

| Farbzahlen von Alkylpolyglucosid-Pasten* | | | | | | |
|---|---|---|---|---|---|---|
| **Bsp.** | **Antioxidantien** | **c(An) ppm** | **V** | **Farbzahl [Gardner]** | | |
| | | | | **24 h** | **2 w** | **4 w** |
| 1 | Tocopherol | 1000 | 1 | 2 | 2 | 3 |
| 2 | Tocopherol | 500 | 2 | 2 | 2 | 2 |
| 3 | BHT | 500 | 1 | 2 | 2 | 2 |
| 4 | BHA | 500 | 1 | 2 | 2 | 2 |
| 5 | Ascorbylpalmitat | 500 | 1 | 3 | 3 | 4 |
| 6 | Ascorbinsäure | 500 | 1 | 3 | 3 | 4 |
| 7 | Citronensäure | 500 | 1 | 3 | 3 | 5 |
| 8 | Ascorbinsäure + Citronensäure | 400 100 | 1 | 2 | 2 | 3 |
| 9 | Natriumnitrit | 50 | 1 | 3 | 3 | 3 |
| V1 | ohne | - | - | 15 | 20 | 20 |
| V2 | Tocopherol | 1000 | - | 8 | 12 | 15 |

| | | | | | | |
|---|---|---|---|---|---|---|
| *) 2-cm-Küvette | | | | | | |

### II. Herstellung einer Alkylsulfat-Paste

In einem kontinuierlich arbeitenden Fallfilmreaktor (Länge 120 cm, Querschnitt 1 cm, Eduktdurchsatz 600 g/h) mit Mantelkühlung und seitlicher SO₃-Begasung wurden 5,0 mol einer Fettalkoholmischung gemäß Produkt A mit einem Schmelzpunkt von 43°C bei 55°C mit 5,35 mol gasförmigem Schwefeltrioxid (Alkohol : SO₃ = 1 : 1,07) zur Reaktion gebracht. Das saure Reaktionsgemisch wurde kontinuierlich in 25 gew.-%ige wäßrige Natriumhydroxidlösung eingetragen, dabei neutralisiert, mit dem Antioxidants versetzt, anschließend mit 2 Gew.-% Wasserstoffperoxid in Form einer 35 Gew.-%igen wäßrigen Lösung gebleicht und schließlich auf einen pH-Wert von 10,9 eingestellt.

In Tabelle 2 sind die Farbzahlen nach Lagerung über 24 h sowie 2 bzw. 4 Wochen bei 70°C in Abhängigkeit von den eingesetzten Antioxidantien wiedergegeben.

**Tabelle 2**

| Farbzahlen von Alkylsulfat-Pasten* | | | | | |
|---|---|---|---|---|---|
| **Bsp.** | **Antioxidantien** | **c(An) ppm** | **Farbzahl [Klett]** | | |
| | | | **24 h** | **2 w** | **4 w** |
| 10 | Tocopherol | 500 | 11 | 15 | 21 |
| 11 | BHT | 500 | 14 | 15 | 22 |
| 12 | BHA | 500 | 12 | 15 | 22 |
| 13 | Ascorbylpalmitat | 500 | 14 | 15 | 23 |
| 14 | Ascorbinsäure | 500 | 14 | 15 | 24 |
| 15 | Citronensäure | 500 | 14 | 16 | 25 |
| 16 | Ascorbinsäure + Citronensäure | 400 100 | 12 | 15 | 23 |
| V2 | ohne | - | 42 | 57 | 78 |

| | | | | | |
|---|---|---|---|---|---|
| Legende: c(An) = Konzentration Antioxidantien *) 4-cm-Küvette | | | | | |

## Patentansprüche

1. Verfahren zur Herstellung hellfarbiger Tenside , **dadurch gekennzeichnet,** daß man die Tenside vor der Bleiche durch Zugabe von Antioxidantien stabilisiert.

2. Verfahren zur Herstellung von hellfarbigen Alkyl- und/ oder Alkenyloligoglykosiden durch sauer katalysierte Acetalisierung von Zuckern mit Fettalkoholen, Neutralisation, destillatives Abtrennen überschüssigen Fettalkohols, Anpasten mit Wasser und alkalische Bleiche, **dadurch gekennzeichnet,** daß man die Glycoside vor der Bleicne durch Zugabe von Antioxidantien stabiisiert.

3. Verfahren zur Herstellung von hellfarbigen wäßrigen Alkyl- und/oder Alkenylsulfaten durch Sulfatierung von Fettalkoholen, Neutralisation mit wäßrigen Basen und Bleiche, **dadurch gekennzeichnet,** daß man die Sulfate vor der Bleiche durch Zugabe von Antioxidantien stabilisiert.

4. Verfahren nach den Ansprüchen 1 bis 3, **dadurch gekennzeichnet,** daß man Antioxidantien einsetzt, die ausgewählt sind aus der Gruppe, die von Tocopherol, tert. Butylhydroxytoluol (BHT), Di-tert.Butylhydroxyanisol (BHA), Ascorbinsäure, Ascorbinpalmitat, Citronensäure und/oder Alkalinitriten gebildet wird.

5. Verfahren nach den Ansprüchen 1 bis 4, **dadurch gekennzeichnet,** daß man die Antioxidantien in Konzentrationen von 5 bis 2000 ppm - bezogen auf die Summe der Einsatzstoffe - einsetzt.

## Claims

1. A process for the production of light-coloured surfactants, characterized in that the surfactants are stabilized before bleaching by addition of antioxidants.

2. A process for the production of light-coloured alkyl and/or alkenyl oligoglycosides by acid-catalyzed acetalization of sugars with fatty alcohols, neutralization, removal of excess fatty alcohol by distillation, formation of a paste with water and alkaline bleaching, characterized in that the glycosides are stabilized before bleaching by addition of antioxidants.

3. A process for the production of light-coloured aqueous alkyl and/or alkenylsulfates by sulfation of fatty alcohols, neutralization with aqueous bases and bleaching, characterized in that the sulfates are stabilized before bleaching by addition of antioxidants.

4. A process as claimed in claims 1 to 3, characterized in that antioxidants selected from the group consisting of tocopherol, tert.butyl hydroxytoluene (BHT), di-tert.butyl hydroxyanisole (BHA), ascorbic acid, ascorbyl palmitate, citric acid and/or alkali metal nitrites are used.

5. A process as claimed in claims 1 to 4, characterized in that the antioxidants are used in concentrations of 5 to 2,000 ppm, based on the sum of the starting materials.

## Revendications

1. Procédé de production d'agents tensioactifs de couleur claire,
caractérisé en ce qu'
on stabilise les agents tensioactifs avant le blanchiment par addition d'antioxydants.

2. Procédé de production d'oligoglycosides d'alkyle et/ou d'alkényl de couleur claire par acétalysation catalysée de manière acide de sucres avec des alcools gras, neutralisation, séparation distillative d'alcool gras en excès, mise en pâte avec de l'eau et d'un produit de blanchiment alcalin,
caractérisé en ce que
les glycosides sont stabilisés avant le blanchiment par addition d'antioxydants.

3. Procédé de production d'oligoglycosides d'alkyle et/ou d'alkényl de couleur claire par sulfatation d'alcools gras, neutralisation avec des bases aqueuses et des agents de blanchiment,
caractérisé en ce qu'
on stabilise les sulfates avant le blanchiment par addition des antioxydants.

4. Procédé selon les revendications 1 à 3,
caractérisé en ce qu'
on utilise des antioxydants qui sont choisis dans le groupe qui est formé de tocophérol, tert-butylhydroxytoluène (BHT), di-tert-butylhydroxyanisol (BHA), acide ascorbique, palmitate ascorbique, acide citrique et/ou nitrites alcalins.

5. Procédé selon les revendications 1 à 4,
caractérisé en ce qu'
on utilise les antioxydants dans des concentrations de 5 à 2000 ppm par rapport à la somme des substances introduites.
